# EUROPEAN PATENT APPLICATION

(11) **EP 4 631 437 A1**
(43) Date of publication of application: **15.10.2025**
(21) Application number: 24174581.9
(22) Date of filing: 07.05.2024
(51) Int. Cl.: A61B 8/08, A61B 8/00

(54) **ULTRASOUND ACQUISITION GUIDANCE**

(30) Priority: 11.04.2024 US 202463632650 P
(71) Applicant: Koninklijke Philips N.V., 5656 AG Eindhoven (NL)
(72) Inventor: WAECHTER-STEHLE, Irina, Eindhoven (NL); GESSERT, Nils Thorben, Eindhoven (NL); WEHLE, Simon, 5656AG Eindhoven (NL); SADEGHI, Seyedali, Eindhoven (NL); CHAUDHARI, Ashish Motiram, Eindhoven (NL); GOOßEN, Andre, Eindhoven (NL)
(74) Representative: Philips Intellectual Property & Standards

(57) **Abstract**

A method and system are provided for image acquisition guidance. The method is based on utilizing a reference image in a database and computing a registration offset between a live image view being acquired by an operator and the reference image. To improve consistency of image acquisition across different clinical applications, the method further comprises receiving an indication of a target image-based operation which is to be performed using the image being acquired by the operator and identifying a reference from a reference image store which meets a pre-defined suitability criterion for performing that operation. For example, this may comprise identifying an image in the store which has a highest value of a suitability score associated with suitability of the image for performing the given image based operation.

## Description

### FIELD OF THE INVENTION

This invention relates to ultrasound acquisition method, in particular to a method for ultrasound acquisition guidance.

### BACKGROUND OF THE INVENTION

Ultrasound imaging can be used in a variety of clinical settings to evaluate patients (e.g. in an intensive care setting, in an operating room and, in a general ward).

Ultrasound image acquisition may be performed for a patient at multiple time points, either over a single short-term monitoring period (e.g. during surgery), or over a long term period. In such contexts, acquisition of consistent image views of an anatomy of interest is important, in order to allow for reliable clinical comparison over time.

By way of illustration, two example modes of ultrasound imaging which are used to ultrasonically evaluate patients are Transthoracic echocardiography (TTE) and transesophageal echocardiography (TEE). TTE is non-invasive and therefore has a wider range of use than TEE. It can be used outside of emergency settings. It also carries a lower clinical risk to the patient (compared to TEE) and can be performed by operators with lower skill levels due to its non-invasive nature. However, it can be more difficult to acquire the correct views of a given anatomy using TTE than TEE, since the operator needs to place the probe in exactly the correct position on the chest (compared with TEE where the esophagus largely constrains the probe placement).

Ultrasound imaging can be used to monitor cardiac function during surgery. For example, the use of TEE to monitor cardiac function is increasing in popularity in clinical settings, particularly in cases where other modes such as TTE is difficult to perform due to patient habitus, external chest injuries, other external medical equipment, or patient dressings. For example, to view the left ventricle, a TEE probe may be navigated to the mid-esophageal position. If a clinician requires a view of the heart at any time during the surgery, the probe is manually manipulated so as to acquire the desired view.

The use ultrasound for continual monitoring of the heart (e.g. in an intensive care environment or during surgery) has been proposed within the medical community, and is a current area of technical development. Ultrasound imaging in this context is useful not only for acquiring and viewing imagery of the patient anatomy, e.g. the dynamic motion of the heart, but also for obtaining quantitative measurements of physiological parameters, e.g. hemodynamic parameters such as left ventricular (LV) volume, stroke volume (SV), cardiac output (CO), LV Ejection Fraction (LVEF). A trend pattern of these parameters can be tracked over time and used to inform patient treatment.

It is known for example to apply model-based segmentation to acquired ultrasound imagery in order to obtain quantitative measurements. For example, automatic segmentation of one or more chambers of the heart can be performed. Measurements of hemodynamic parameters can be obtained through applying model-based segmentation on an end-diastolic (ED) ultrasound image frame to detect heart chamber boundaries, followed by 2D or 3D tracking of the segmentation boundaries over the other frames of the cardiac cycle.

For 2D imaging, geometric assumptions can be made to extrapolate from acquired single-plane or bi-plane information, to generate measurement information for 3D objects or features, e.g. heart chamber volumes. Example algorithms include modified Simpson's rule, ellipsoidal model using single-plane, bi-plane or unidimensional data, or the hemisphere-cylinder model based on bi-plane data.

For further details on these segmentation methods, reference is made to: Kosaraju A, Makaryus AN. Left Ventricular Ejection Fraction. In: StatPearls [Internet]. Treasure Island (FL): StatPearls Publishing; 2019 Jan-. Available from: https://www.ncbi.nhn.nih.gov/books/NBK459131/.

Longitudinal ultrasound monitoring of a patient over a continuous time period (e.g. over hours) can be challenging because the probe position can drift over time. In an intensive care environment, measurements might be taken by multiple clinicians or specialists at different points in time. Measurements may also be desired at times when a cardiologist, intensivist or other specialist is absent. Furthermore, TTE imaging, by way of example, also presents its own challenges since the same correct view has to be obtained on each occasion from scratch.

Additionally, when generating longitudinal trends for a given measurement (e.g. SV in the case of cardiac imaging), it is important to ensure consistency in the image data provided to the measurement tool. If the image data provided varies in the particular view depicted for example, measurement errors associated with the inconsistent image acquisition can occur, and thus can interfere with the interpretation of longitudinal trends. This may result in inappropriate additions or changes to the medical interventions provided to the patient (e.g., fluid status change, vasopressors, inotropes). This is especially true in 2D imaging approaches, where the possibility of foreshortening (in which the acquired ultrasound plane does not cut through the true apex of the left ventricle) is a significant challenge.

It would be advantageous to provide a solution which enables acquisition of more consistent image data across time.

### SUMMARY OF THE INVENTION

The invention is defined by the claims.

According to examples in accordance with an aspect of the invention, there is provided a method for ultrasound image acquisition guidance.

The method comprises obtaining an indication of a target image-based operation to be performed during an ultrasound scan with an ultrasound imaging apparatus.

The method further comprises identifying, in an image datastore, a reference image associated with the target image-based operation to be performed. The reference image may for example be an image acquired in a previous ultrasound scan with the same or a different imaging apparatus. The identifying may comprise identifying the reference image as meeting a pre-defined suitability criterion indicative of suitability for performing the target image-based operation.

The method further comprises acquiring live ultrasound image data at the ultrasound imaging apparatus.

The method further comprises registering a current image of the live ultrasound image data with the reference image.

The method may further comprise, based on the registration, providing acquisition guidance on a user interface to guide a user in adjusting the acquisition of the live ultrasound imaging data to improve a view represented in the current image.

The proposed system aims to provide view acquisition guidance in real time to an ultrasound operator based on registration between a live image view and a target (reference) image view. Embodiments of the invention are based on the concept of introducing a selection step for selecting the reference image to be used, based on an optimization criterion. In particular, the method involves identifying a reference image which meets a suitability criterion, where the suitability criterion depends upon the target task for which the acquired image is to be used. In this way, consistency in image view acquisition is improved and in a way that is more fine-tuned to the particular application for which the acquired image is to be used.

Improving a view represented in the current image may comprise improving a match a view represented in the current image and a view represented in the reference image. Additionally or alternatively, improving a view represented in the current image may comprise improving an image quality of the view represented in the current image.

In some embodiments, the method may be performed in the context of a distributed system comprising multiple ultrasound acquisition devices, each operatively communicable with the datastore.

Embodiments of the invention assist in improving consistency of imaging for purposes of performing one or more different image-based operations. This may assist for example in improving consistency of scanning in longitudinal examinations for a single patient.

The images may be 2D images or 3D images.

In some embodiments, the suitability criterion comprises a suitability score for performing the target image-based operation associated with the image meeting a pre-determined threshold value. In some of these embodiments, the suitability score may be an image quality score representative of an image quality of the image. In that case, the suitability criterion may comprise the image quality score of the image meeting a pre-determined image quality threshold value.

In some embodiments, the reference image is labelled in the datastore with a suitability score for performing the target image-based operation. In this case, the suitability score associated with the image may be determined by reading the suitability score label from the database. Additionally or alternatively, the suitability score associated with the image may be determined by applying a suitability analysis module to the reference image configured to generate a suitability score. The suitability analysis module may be applied in real time or on-the-fly in some embodiments.

In some embodiments, the reference image may be labelled in the datastore with a suitability score for performing the target image-based operation, and wherein the suitability criterion comprises the suitability score meeting a pre-defined threshold value.

In some embodiments, the image datastore stores a plurality of previously acquired images, acquired in one or more previous ultrasound scans, and each previously acquired image being labelled with a view represented by the image.

In some embodiments, the method may comprise detecting a view represented by the current image of the live ultrasound image data using a view detection module. The method may comprise identifying an image from the image datastore which meets the suitability criterion for the target image-based operation and which additionally represents a matching view to the currently acquired image. In this way, the system automatically selects a reference image for guidance image acquisition which depicts a corresponding anatomical view to the one which the user is trying to acquire. For example, the system may automatically detect one of a set of standardized image views (e.g. from an imaging protocol) which the user is seeking to acquire and automatically selects a reference image which represents the same standardized image view.

In some embodiments, each image in the datastore is associated with a suitability score for performing the target image-based operation and wherein the identifying the image from the image datastore comprises identifying an image from the image datastore having the highest suitability score for the target image-based operation.

In some embodiments, the identifying the image from the image datastore comprises identifying an image from the image datastore having the highest suitability score for the target image-based operation and which additionally represents a matching view to the currently acquired image.

The method may comprise determining the score either by reading a score label attached to the image from the database or by applying a suitability analysis module for example.

In some embodiments, at least a subset of the images stored in the image datastore are each labelled with suitability scores for more than one different possible image-based operation. In this way, the same reference images may be used for different image-based tasks or applications. Likewise, the suitability analysis module may permit assessing suitability of input images for different possible image-based operations.

In some embodiments, the aforementioned view detection module may comprise a convolutional neural network trained to predict a view of an anatomy depicted in an image.

In some embodiments, the method comprises computing the suitability scores for the images in the image datastore by application of a suitability analysis module which utilizes a trained convolutional neural network trained using prior images of an anatomy which have been labelled with suitability scores for at least one image-based operation.

In some embodiments, the method may comprise computing the suitability scores in advance and storing these in the image datastore, or computing the suitability scores on the fly during execution of the method.

In some embodiments, the method further comprises performing the image-based operation on the current image of the live ultrasound image data.

In some embodiments, the target image-based operation comprises acquiring and storing an image representing a pre-defined target view of an anatomical structure. The target image-based operation may additionally comprise storing said image. In these embodiments, the suitability criterion/score may be an image quality score representative of an image quality of the reference image. The image quality score may reflect (e.g., may be based on) one or more pre-defined aspects of image quality, such as a level of image noise, a level of image blur, and/or a visibility of one or more pre-defined anatomical features.

Additionally or alternatively, the suitability criterion/score may comprises a view suitability criterion/score representative of a quality of match between the view represented by the reference image and a pre-defined target view of the anatomical structure.

In some embodiments, the performing the image-based operation comprises storing the current image of the live ultrasound data into a memory.

In some embodiments, the image-based operation comprises acquiring a target measurement of an anatomical structure depicted in the current image. In this case, the suitability criterion may represent a suitability of the reference image for use in acquiring the target measurement.

In some embodiments, the performing the image-based operation comprises applying a measurement quantification module to the current image of the live ultrasound image data to acquire the target measurement from the current image.

In some embodiments, the providing the acquisition guidance comprises displaying an overlay of one or more reference points of the reference image atop the current image, for example wherein the reference points represent anatomical landmarks.

In some embodiments, the providing the acquisition guidance comprises displaying an overlay of a field-of-view boundary of the reference image atop the current image, based on the registration. This is sometimes referred to as a bounding box overlay.

In some embodiments, the aforementioned registration comprises determining a rotation offset and a translation offset between an anatomy depicted in the current image of the live ultrasound image data and the same anatomy depicted in the reference image.

In some embodiments, the displaying an overlay of a field-of-view boundary of the reference image comprises estimating a location of a field-of-view boundary of the reference image relative to the current image based on the rotation offset and translation offset, and rendering the overlay of the field of view boundary based on the estimate.

In some embodiments, the reference image is labelled in the database with a set of acquisition parameters of an ultrasound imaging apparatus used to acquire the reference image, and wherein the method comprises configuring the ultrasound imaging system based on the set of acquisition parameters used to acquire the reference image. In this way, the system provides guidance on view alignment to the operator as well as matching the acquisition parameters of the imaging system to those used to acquire the reference image. Thus, the matching of the live image to the target image is achieved on two levels: the level of the anatomical view depicted in the image, and the level of the imaging parameters/settings of the imaging system.

In some embodiments, the reference image is further labelled with a set of acquisition parameters of an ultrasound imaging apparatus used to acquire the reference image, and wherein the method comprises generating a guidance prompt on a user interface indicative of the set of acquisition parameters used to acquire the reference image. In this way, the user is provided with the information necessary to manually configure the acquisition settings to match those which were used to acquire the reference image.

In some embodiments, the method may comprise a step or procedure of compiling the image datastore. This might additionally or alternatively be provided as a separate aspect of the invention. The procedure may comprise obtaining a plurality of reference images acquired in one or more previous ultrasound scans. Optionally, it may comprise obtaining an indication of acquisition parameters of an ultrasound imaging apparatus used to acquire each of the plurality of reference images. The procedure may further comprise, for each of the plurality of reference images: detecting a view represented by the image using a view detection module; determining at least one suitability score for the image for performing at least one target image-based operation; and storing the reference image in the image datastore labelled with an indication of the detected view represented by the image and the determined at least one suitability score. Optionally, the image may be further labelled with the acquisition parameters of the ultrasound imaging apparatus used to acquire the reference image.

In some embodiments, the view detection module comprises a trained neural network.

Another aspect of the invention is a computer program product comprising machine-executable code configured, when run on a processor, to cause the processor to perform a method in accordance with any example or embodiment described in this disclosure or in accordance with any claim of this application.

Another aspect of the invention is a processing device comprising one or more processors configured to perform a method in accordance with any example or embodiment described in this disclosure or in accordance with any claim of this application.

Another aspect of the invention is a system comprising: the processing device outlined above; and an ultrasound acquisition apparatus operatively coupled with the processing device.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 outlines steps of an example method in accordance with one or more embodiments of the invention;
Fig. 2 is a block diagram of an example processing device and system in accordance with one or more embodiments of the invention;
Fig. 3 is a block diagram illustrating an example processing flow for one set of embodiments in which the target image-based operation is image acquisition and storage;
Fig. 4 is a block diagram illustrating an example processing flow for one set of embodiments in which the target image-based operation is an image-based measurement;
Fig. 5 shows an example guidance output provided by the system comprising a field of view outline overlay on the live ultrasound image view;
Fig. 6 shows an example guidance output provided by the system for guiding 3D image acquisition, comprising guidance markers indicative of 3D alignment between anatomical key points of the imaged anatomy in the reference image compared with the live image;
Fig. 7 shows an example distributed imaging system within which embodiments of the present invention may operate; and
Fig. 8 shows a further example distributed imaging system within which embodiments of the present invention may operate.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method and system for image acquisition guidance, based on utilizing a reference image in a database and computing a registration offset between a live image view being acquired by an operator and the reference image. To improve consistency of image acquisition across different clinical applications, the method further comprises receiving an indication of a target image-based operation which is to be performed using the image being acquired by the operator and identifying a reference from a reference image store which meets a pre-defined suitability criterion for performing that operation. For example, this may comprise identifying an image in the store which has a highest value of a suitability score associated with suitability of the image for performing the given image based operation.

An aim in accordance with embodiments of the present invention is to enable more consistent medical image scanning, for example for longitudinal examinations of a same patient. Consistent scanning refers to acquiring a consistent view of an anatomy of interest in each image which is acquired. For example, for longitudinal examination, for subsequent images to be comparable, it is important that each image depicts as close as possible to an identical view of the anatomy of interest.

Furthermore, consistency of image acquisition is important even in single examination applications, in order for obtained results to provide for accurate interpretation by a human clinician or an automated diagnostic algorithm. For example, it is common to apply analytics algorithms which automatically quantify one or more anatomical measurements pertaining to the imaged anatomy. For these purposes, it may also be important that the acquired image is correctly aligned with one of a set of standardized image views defined by an imaging protocol.

Consistent scanning is challenging, particularly for inexperienced users.

Embodiments of the present invention provide a system and method for guiding image acquisition to ensure greater consistency, and in a way that is flexible to different tasks which are to be performed using the acquired images. In particular, depending upon the task for which the image is to be used, different reference images are utilized to guide real-time image acquisition. The selection of a reference image is guided by suitability criteria which may be evaluated in advance for each available reference image or may be determined on the fly.

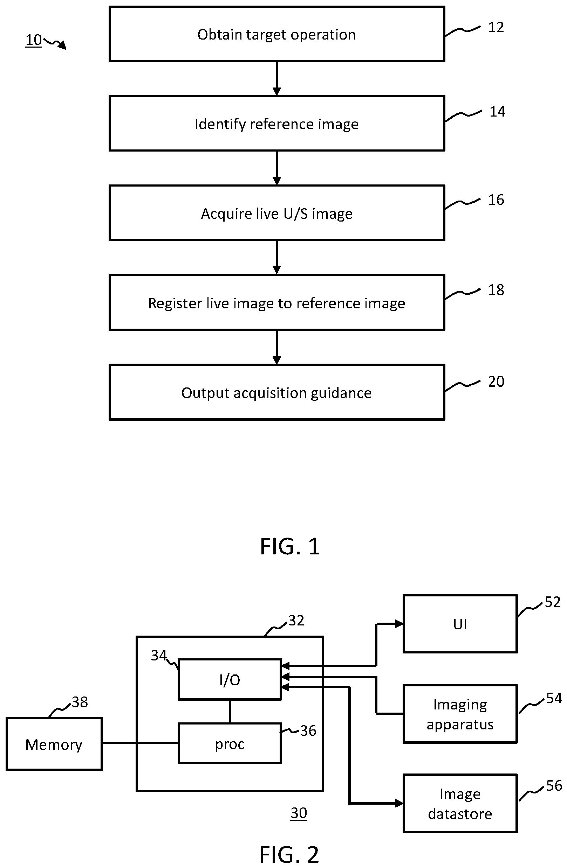

Fig. 1 outlines in block diagram form steps of an example method according to one or more embodiments. The steps will be recited in summary, before being explained further in the form of example embodiments.

Provided is a method 10 for medical image acquisition guidance, for example ultrasound image acquisition.

The method comprises obtaining 12 an indication of a target image-based operation to be performed during an ultrasound scan with an ultrasound imaging apparatus. This may for example be obtained as a user input from a user interface. Alternatively, it might be obtained from a datastore. Alternatively, it might be received as configuration data from another computer, e.g. a patient monitoring station.

The method comprises identifying 14 in an image datastore a reference image, the reference image having been acquired in a previous ultrasound scan with the same or a different imaging apparatus. The identifying may comprise identifying the reference image as meeting a pre-defined suitability criterion indicative of suitability for performing the target image-based operation. In some embodiments, the identifying the reference image may comprise a step of determining whether the reference image meets the suitability criterion. In some embodiments, the suitability criterion is a minimum value of a suitability score. In some embodiments, the method comprises determining a value for the suitability score for the reference image and comparing the determined value against a threshold, where the threshold defines the suitability criterion. The value of the suitability score may be determined in advance for the reference image and stored in the datastore along with the reference image. There may be a plurality of available reference images in the datastore, each labelled with a pre-computed value of the suitability score. In some embodiments, the identifying the reference image comprises selecting the one of the plurality of reference images in the datastore with the highest value for the suitability score for the given image-based operation.

The method 10 further comprises acquiring 16 live ultrasound image data at the ultrasound imaging apparatus.

The method 10 further comprises registering 18 a current image of the live ultrasound image data with the reference image.

The method 10 further comprises, based on the registration, providing 20 acquisition guidance on a user interface to guide a user in adjusting the acquisition of the live ultrasound imaging data to improve a match between a view represented in the current image and a view represented by the reference image.

Optionally, in some embodiments, the reference image is labelled in the database with a set of acquisition parameters of an ultrasound imaging apparatus used to acquire the reference image. The method may further comprise configuring the ultrasound imaging system based on the set of acquisition parameters used to acquire the reference image. Additionally or alternatively, the method may comprise generating a guidance prompt on a user interface indicative of the set of acquisition parameters used to acquire the reference image. In this way, consistency in the image acquisition is improved both in terms of the view represented by the image and the imaging settings used to acquire the image. A prompt may be presented on the user interface requesting confirmation by the user of the set of acquisition parameters used to acquire the reference image. The user interface may permit the user to confirm the parameters or reject the parameters. If confirmed, the acquisition parameters are implemented on the imaging apparatus.

As noted above, the method can also be embodied in hardware form, for example in the form of a processing device which is configured to carry out a method in accordance with any example or embodiment described in this document, or in accordance with any claim of this application.

To further aid understanding, Fig. 2 presents a schematic representation of an example processing device 32 configured to execute a method in accordance with one or more embodiments of the invention. The processing device is shown in the context of a system 30 which comprises the processing device. The processing device alone represents an aspect of the invention. The system 30 is another aspect of the invention. The provided system need not comprise all the illustrated hardware components; it may comprise only a subset of them.

The processing device 32 comprises one or more processors 36 configured to perform a method in accordance with that outlined above, or in accordance with any embodiment described in this document or any claim of this application. In the illustrated example, the processing device further comprises an input/output 34 or communication interface.

In the illustrated example of Fig. 2, the system 30 further comprises a user interface 52.

In the illustrated example of Fig. 2, the system 30 further comprises an ultrasound acquisition or imaging apparatus 54 for acquiring ultrasound imaging data. The imaging apparatus 54 may be for acquiring 2D ultrasound data and/or 3D ultrasound data.

In the illustrated example of Fig. 2, the system further comprises an image datastore 56. The image datastore 56 stores one or more reference images, for example acquired in one or more previous ultrasound scans. Preferably, the image datastore stores a plurality of previously acquired images, each acquired in one or more previous ultrasound scans. In some embodiments, each image in the image datastore may be labelled with a view represented by the image. In some embodiments, the method 10 comprises detecting a view represented by the current image of the live ultrasound image data using a view detection module. In some embodiments, the method further comprises identifying an image from the image datastore which meets the suitability criterion for the target image-based operation and which represents a matching view to the currently acquired image.

In some embodiments, the suitability criterion comprises a suitability score for performing the target image-based operation associated with the image meeting a pre-determined threshold value. In some embodiments, each image in the image datastore 56 is associated with a suitability score for performing the target image-based operation and wherein the identifying the image from the image datastore comprises identifying an image from the image datastore having the highest suitability score for the target image-based operation and which represents a matching view to the currently acquired image. The method 10 may comprise determining the suitability score either by reading a score label attached to the image from the database or by applying a suitability analysis module.

In some embodiments, at least a subset of the images stored in the image datastore are each labelled with suitability scores for more than one different possible image-based operation. In this way, the system permits selection from a same image datastore of a suitable reference image for guiding acquisition of an image for any of a range of possible applications.

The system 30 may further comprise a memory 38 for storing computer program code (i.e. computer-executable code) which is configured for causing the one or more processors 36 of the processing unit 32 to perform the method as outlined above, or in accordance with any embodiment described in this disclosure, or in accordance with any claim.

As mentioned previously, the invention can also be embodied in software form. Thus another aspect of the invention is a computer program product comprising computer program code configured, when run on a processor, to cause the processor to perform a method in accordance with any example or embodiment of the invention described in this document, or in accordance with any claim of this patent application.

As mentioned previously, embodiments of the invention involve identifying a reference image from the image datastore which meets a suitability criterion for performing an image-based operation.

In some embodiments, the target image-based operation comprises acquiring and storing an image representing a pre-defined target view of an anatomical structure.

For example, the image-based operation comprises storing the current image of the live ultrasound data into a memory.

Here, the suitability criterion/score may comprise a view quality criterion/score representative of a quality of the reference image. For example, the quality score may reflect one or more aspects of image quality such as degree of image noise, degree of image blur, and/or visibility of one or more pre-defined anatomical features.

Additionally or alternatively, the suitability criterion/score may comprise a view suitability criterion/score representative of a quality of match between the view represented by the reference image and the pre-defined target view of the anatomical structure.

In some embodiments, for each acquisition, the system may be configured to automatically determine a view which the user is seeking to scan using a view detection module. The output of the view detection module may be a view ID or other identifier of the target view. Optionally, the image datastore 56 may be searched with the view identifier to find a reference image matching the target view, and meeting a pre-defined suitability criterion for the target image-based operation. The image datastore may be searched to find the image with best image quality of the same view of a previous exam. Optionally, the acquisition settings used to acquire the previous reference image may be stored in the datastore and retrieved along with the image. If the settings of the previous acquisition are different to the current settings of the imaging apparatus, the current settings may be changed to match those used to acquire the previous image.

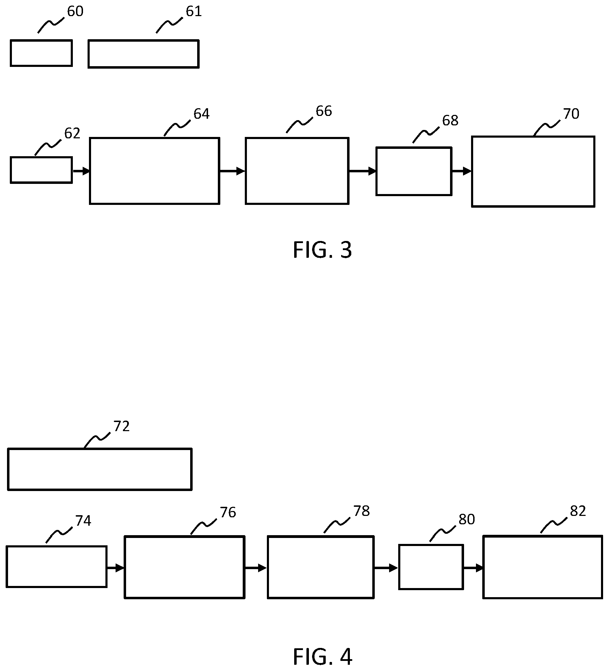

Fig. 3 shows a flow diagram illustrating an example implementation in accordance with this set of embodiments.

For each reference image stored in the image datastore 56, an anatomical view depicted by the image is detected using a view detection module, and an image quality is determined, and both are stored as metadata of the image in the image datastore. The anatomical view is represented for example by a view ID label 60, and the image quality may be represented by an image quality label 61. These properties of each image may be determined in advance for each image and stored in the datastore, or for at least a subset of the images the properties may be determined in real time during use of the system. Optionally, a set of acquisition parameters used to acquire each reference image is additionally stored as metadata of the respective reference images.

In operation of the system, the user controls an imaging probe of the ultrasound imaging apparatus to configure a positioning of the imaging field of view within the anatomy being imaged. The system processes a current image frame with a view detection module to determine an anatomical view 62 represented by the current image frame. The system may automatically apply the view detection module at regular time intervals or at regular image frame intervals, or for every image frame. Alternatively, the user interface may permit the user to manually trigger the view detection module. The detected image view may take the form of a view ID 62. The system proceeds by searching the image datastore 56 for a reference image which represents a same image view, and which meets a pre-defined suitability criterion, where the suitability criterion is a threshold image quality score. For example, the reference image having the highest image quality score depicting a same view ID as the current live image may be identified.

Once a suitable reference image has been identified, optionally, the system may further read from the metadata of the image a set of acquisition parameters 66 used to acquire the reference image and may adjust the current acquisition parameters of the imaging apparatus to match those associated with the reference image.

The system is further configured to register 68 the current image to the identified reference image. Based on the registration, the system determines and displays guidance information 70 for guiding the user of the imaging apparatus in acquiring an image view which better matches that of the reference image. For example, the registration may yield a registration offset between the identified reference image and the current live image. The guidance information may be information for guiding a user to reduce the registration offset. For example, the guidance information may take the form of instructions for the user for changing a position and/or pose of the ultrasound imaging probe to improve the alignment between the current live image and the reference image.

According to one or more further embodiments, the image-based operation comprises acquiring a target measurement of an anatomical structure depicted in the current image. For example, performing the image-based operation comprises applying a measurement quantification module to the current image of the live ultrasound image data to acquire the target measurement from the current image.

Here, the suitability criterion may represent a suitability of the reference image for use in acquiring the target measurement. In other words, for this set of embodiments, the suitability criterion is not generic image quality but the suitability of the image for a certain measurement.

For this set of embodiments, the user may provide a user input via the user interface indicative of a target measurement to be acquired. The image datastore is searched for a reference image which meets a pre-determined suitability criterion for use in acquiring the specified measurement. This may be a suitability score for acquiring the specified measurement. Optionally, the acquisition settings used to acquire the reference image are also retrieved and the current settings of the imaging system are adjusted to match those of reference image.

Fig. 4 shows a flow diagram of an example implementation in accordance with this set of embodiments.

Each image in the image datastore is labelled with suitability score 72 indicative of suitability of the reference image for use in acquiring one or more target measurements, where the one or more target measurements include at least the target measurement which the user is acquiring.

During operation, an indication is acquired 74 of a target measurement which the user is planning to acquire. This information may be received as a user input from a user interface. Alternatively, this information can be inferred automatically based on: detecting an anatomical view represented by a current live image frame and inferring a target measurement from the anatomical view. For example, only a limited set of measurements may be possible for any given anatomical view, and thus a target measurement can be inferred from the current view.

Following this, the system proceeds by searching the image datastore for the reference image which has the highest value of the suitability score for the user-input target measurement. The identified reference image is retrieved 78 from the datastore. Optionally, each reference image in the datastore may be labelled with a set of acquisition parameters used to acquire the reference image. Optionally, the system may further read from the metadata of the image a set of acquisition parameters used to acquire the reference image and may adjust the current acquisition parameters of the imaging apparatus to match those associated with the reference image. The system is further configured to register 80 the current image to the reference image. Based on the registration, the system determines and displays guidance information 82 for guiding the user of the imaging apparatus in acquiring an image view which better matches that of the reference image. Optionally, the image suitability score associated with the identified reference image is displayed using the user interface in combination with the guidance information.

Optionally, in accordance with any of the above embodiments, a value of the relevant image suitability criterion or score may be calculated for the current live image and may be displayed in real time as the user controls the image acquisition system. This helps a user to understand quantitatively how closely the current image matches the reference image.

As discussed above, embodiments of the invention involve identifying a reference image which meets a pre-determined suitability criterion indicative of suitability for performing the target image-based operation.

Options for different suitability criteria and means for their computation will now be discussed in more detail.

The suitability criterion may comprise a suitability score associated with the reference image meeting a threshold.

In some embodiments, the method comprises computing suitability scores for images in the image datastore by application of a suitability analysis module which utilises a trained convolutional neural network trained using prior images of an anatomy which have been labelled with suitability scores for at least one image-based operation.

The method may comprise computing the suitability scores in advance and storing these in the database, or computing the suitability scores on the fly during execution of the method.

By way of example, where the image-based operation is acquiring and storing an anatomical image, the suitability score may be indicative of image quality of the image. In this case, the suitability analysis module may be an image quality assessment module.

In some embodiments, the image quality assessment module may comprise an artificial neural network, for example a convolutional neural network. The network may be trained using a training dataset of e.g. several thousand frames, each annotated according to an image quality indicator or metric. The annotations may be added manually by a human annotator in some examples. Alternatively, image quality of each training image could be computed by another image quality assessment algorithm. The image quality annotations may be indicative of any of a variety of possible aspects of image quality, e.g. degree of image noise, degree of image blur, and/or visibility of one or more anatomical features.

The model may be trained in a supervised manner to predict image quality of an input image frame.

Where the image-based operation comprises acquisition of a measurement from the image, a neural network may be trained to assess suitability of an input image for acquiring a particular measurement. For example, a training dataset of images may be compiled, wherein each image is tagged in advance with a suitability score. For example, the suitability score may be determined manually by a user in some examples. Alternatively, each image may be processed with the relevant measurement quantification algorithm and the accuracy of the output measurement evaluated, and wherein the suitability score is determined for each image based on the accuracy of the measurement derived from the image.

According to some embodiments of the invention, the method may further include a preliminary step of compiling the image datastore 56. As discussed above, this may comprise obtaining a plurality of reference images acquired in one or more previous ultrasound scans, and for each of the plurality of reference images:
detecting a view represented by the image using a view detection module; determining at least one suitability score for the image for performing at least one target image-based operation; and storing the reference image in the image datastore labelled with an indication of the detected view represented by the image and the determined at least one suitability score.

At least a subset of the images stored in the image datastore may each be labelled with suitability scores for more than one different possible image-based operation. In this case, the image datastore may store a plurality of previously acquired images, acquired in one or more previous ultrasound scans, and each previously acquired image labelled with a view represented by the image.

In operation, the method may comprise: detecting a view represented by the current image of the live ultrasound image data using a view detection module; and identifying an image from the image datastore which meets the suitability criterion for the target image-based operation and which represents a matching view to the currently acquired image. Each image in the image datastore may be associated with a suitability score for performing the target image-based operation and wherein the identifying the image from the image datastore comprises identifying an image from the image datastore having the highest suitability score for the target image-based operation and which represents a matching view to the currently acquired image.

The method may comprise determining the suitability score of each reference image either by reading from the database a score label associated with the image or by applying a suitability analysis module.

As mentioned above, in some embodiments, the method comprises detecting a view represented by the live ultrasound image data. In this way, a reference image can be identified from the image datastore which best matches the image the user is seeking to acquire. Detection of the view may be performed by a view detection module. In some embodiments, the view detection module may comprise a convolutional neural network trained to predict a view of an anatomy depicted in an image. In some embodiments, the view identification model is a deep learning model (convolutional neural network, CNN), trained in a supervised manner to predict the cardiac view of a single frame. For training the model, a training dataset may be used comprising a plurality of previously acquired images, each labelled according to the anatomy view depicted. For example, the training dataset may comprise several thousand images.

By way of example, the model architecture may be based on architecture VGG (Vision Graphics Group), comprising several stacked convolutional layers, including batch normalization and ReLU activation functions.

As previously mentioned, embodiments of the invention involve registering the identified reference image with the live ultrasound image data.

For the registration of the two images, many different algorithms can be used. Both intensity and feature-based algorithms are possible. As difference metrics, the sum of differences or mutual information can for example be used.

As an alternative, deep learning-based registration can be employed. Here, a deep learning model is trained to estimate the registration parameters that are required to map one image onto the other.

In any scenario, the output of the registration method is preferably the rotation and shifting parameters which define the registration offset between the live ultrasound image and the reference image. This can then be used to generate and display guidance information for guiding the user in adjusting the position of the ultrasound probe to improve a match between the view represented in the reference image and the live ultrasound image. By way of example, the registration information can be used to generate and display a representation of the outline of the field of view of the reference image atop the live ultrasound image view. For example, this represents a position of an outline of the field of view cone of the reference image superposed atop the rendered view of the current acquisition on the user interface display.

Thus, to state this more explicitly, in some embodiments, the registration may comprise determining a rotation offset and a translation offset between an anatomy depicted in the current image of the live ultrasound image data and the same anatomy depicted in the reference image.

In some embodiments, the providing the acquisition guidance comprises displaying an overlay of a field-of-view boundary of the reference image atop the current image, based on the registration. By way of example, the displaying an overlay of a field-of-view boundary of the reference image may comprise estimating a location of a field-of-view boundary of the reference image relative to the current image based on the rotation offset and translation offset, and rendering the overlay of the field of view boundary based on the estimate.

In some embodiments, the registration may be a rigid registration, i.e. assuming a common size and shape of objects depicted in the two images, with translation and rotation position being the only two variables. For example, an affine transformation may not be needed. This is particularly so if the image acquisition parameters have been matched between the reference image and the current image, as discussed above, since then the scale of the two images can be expected to match.

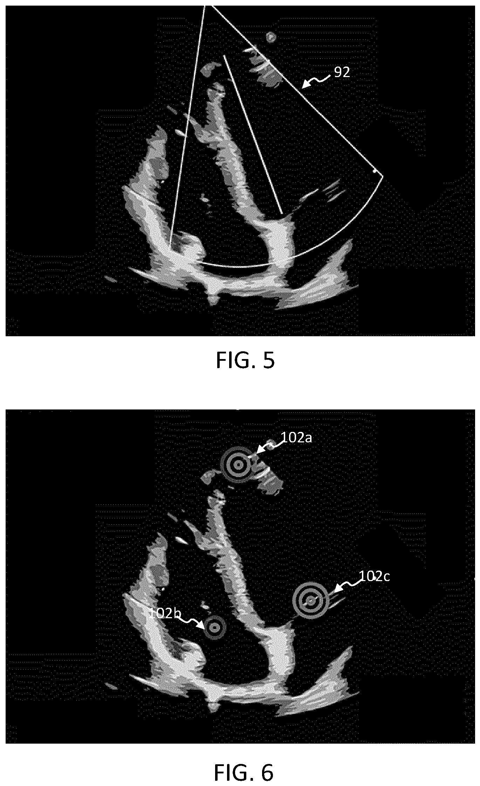

Fig. 5 and Fig. 6 illustrate example guidance indicia which might be presented on a display in accordance with one or more embodiments, based on the registration information.

Fig. 5 shows an example of guidance information in the case where the providing the acquisition guidance comprises displaying an overlay of a field-of-view boundary of the reference image atop the current image, based on the registration. Fig. 5 shows an example overlay of a field of view boundary 92. This has a cone shape in the example of Fig. 5 to reflect the cone-shaped field of view of the ultrasound probe. It can be seen that the field of view boundary is rotated relative to the field of view of the current live image behind the overlay. This provides guidance to the user that rotation of the probe angle is needed to achieve alignment between the current live image and the reference image.

Additionally or alternatively, other indicia may be superposed on the current image frame for guiding the user in acquisition, for example indicative of the location of landmarks, of measurement callipers and/or of contours or edged of the reference image.

Fig. 6 shows an example in which the providing the acquisition guidance comprises displaying an overlay of one or more reference points 102a, 102b, 102c of the reference image atop the current image. For example, the reference points may represent anatomical landmarks. This is a particularly useful guidance mode in the case of 3D imaging. The reference points may provide target alignment markers, wherein alignment of anatomical landmarks in the current live image with the displayed reference points achieves alignment of the view of the live image with the reference image.

In some embodiments, a graphical characteristic of the reference points 102, e.g. a size, shape and/or color, may be controlled to vary as a function of proximity of the live image with exact alignment with the reference image. This may be particularly useful for 3D imaging where alignment between the fields of view of the reference and live images must be performed in three dimensions. For each reference point, a graphical characteristic of the reference point may vary as a function of degree of alignment in at least one dimensional direction between an anatomical landmark of the reference image and a same anatomical landmark of the live image. For example, if the imaging is 3D imaging, the at least one dimensional direction may be a dimensional direction out-of-plane of the 2D plane slice depicted on the user interface display. Thus, the reference points may assist the user to additionally capture depth information in the guidance information. This information is especially useful to circumvent foreshortening and to capture the field of view correctly.

In some embodiments, the method may be performed in the context of a distributed system comprising multiple ultrasound acquisition units, each operatively communicable with the datastore.

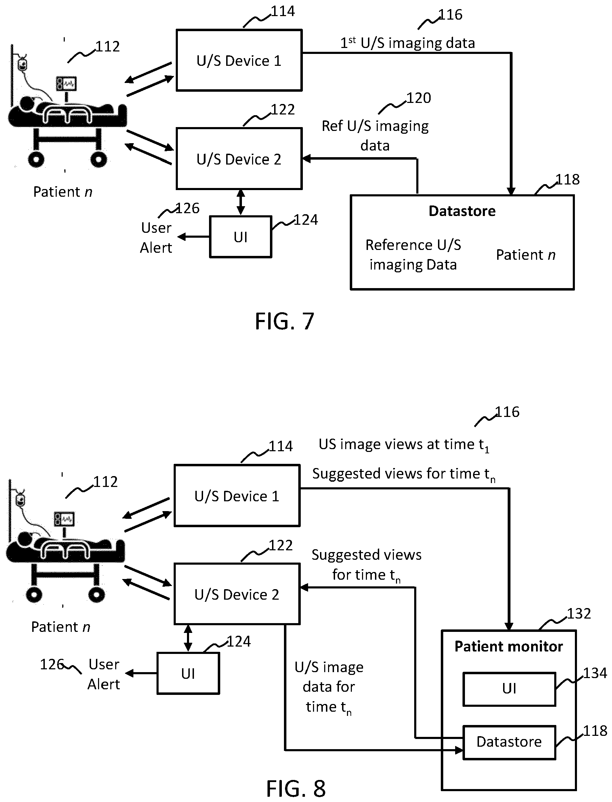

Fig. 7 schematically outlines components of an example system in accordance with an aspect of the invention.

The system comprises one or more ultrasound imaging devices 114, 122. In this example, two ultrasound imaging devices 114, 122 are shown. However, more than two, or only one, is an option in other embodiments. The system further includes a datastore 118 for storing ultrasound imaging data for multiple patients.

The system may be a distributed computing system, meaning that the different components or nodes of the system may be physically isolated from another, but connected in a functional network, for collaborative data acquisition, analysis, storage and user presentation.

The system is for implementing a computer implemented method, for aiding the acquisition of ultrasound imaging data over an extended period of time, which has consistency in the ultrasound image views which are obtained. This computer implemented method forms a further aspect of the present invention. The steps of an example computer-implemented method in accordance with one or more embodiments of the invention will now be outlined in more detail, with reference to components of the system of Fig. 1.

The example method comprises a step of acquiring at a first ultrasound imaging device 114 ultrasound imaging data 116 of a subject 112, including at least one image depicting at least one view of an anatomy of the subject.

The method further comprises storing the first ultrasound imaging data 116 as reference image data in an image datastore 118. In some embodiments, this may be tagged with subject identifier information. In some embodiments, it may be tagged with information indicative of acquisition parameters of the imaging device used to acquire the image. In some embodiments, the method further comprises computing one or more suitability scores for the image indicative of suitability for the image for performing one or more respective image-based operations. This process have been discussed in more detail above. As discussed above, alternatively, the suitability scores can be determined in real time (on the fly) at a later point.

The datastore 118 corresponds to the image datastore 56 referred to previously with reference to Fig. 2. The datastore, in some embodiments, may be a datastore comprised by a patient monitoring subsystem, so that the patient monitoring subsystem acts as a central hub of the system. The patient monitoring system might at the same time be arranged to receive biological sensor data relating to the patient 112, and adapted to display this on a display of a user interface. The display of the user interface 124 may also be controlled to display the acquired first ultrasound imaging data. In some examples, the patient monitoring subsystem, or a separate processing component connected to the patient monitoring subsystem, may be adapted to process the ultrasound imaging data and obtain from it one or more quantitative anatomical and/or physiological measurements, e.g. hemodynamic measurements. The datastore may instead be located elsewhere. It may be a separate dedicated data storage node, e.g. a storage server, e.g. at a central location in the medical institution. It may be a cloud-based data storage means, accessible via an internet connection.

The method may further comprise, retrieving from the datastore 118, at the first ultrasound device 114 or at a second different ultrasound imaging device 122, the reference ultrasound image data. This is done at a later time. As discussed previously, this is based on identifying in the datastore a reference image which meets a pre-defined suitability criterion indicative of suitability for performing a target image-based operation to be performed during an ultrasound scan with one of the ultrasound imaging devices 114, 122. Information indicative of the target image based operation to be performed is obtained for example from a user interface 124 of the system, or from another source such as a database.

As mentioned previously, in some embodiments, each reference image in the image datastore 118 may be already tagged with one or more image suitability scores for use in assessing the suitability criterion, where this is performed in advance of execution of the method. Alternatively, the suitability scores for candidate reference images may be evaluated in real time during acquisition of the live ultrasound image data.

As mentioned above, in some embodiments, the reference images may be tagged with patient identifier information so that a patient's own historical scan images can be used as references. In this case, the identifying the reference image in the datastore may additionally be based on querying the datastore with patient identifier information. For example, an operator at the second ultrasound imaging device 122 inputs the patient identifier information to the second ultrasound imaging device 122 in a setup or initiation step, and this prompts retrieval of the relevant ultrasound image data from the datastore 118. The second imaging device 122 may in fact be the same imaging device as the first (i.e. there is only one imaging device), but being operated at a later time, for example by a different operator to the one who acquired the first ultrasound imaging data 116. Alternatively, the second ultrasound imaging device 122 may be a different imaging device, which may be located at the same physical location, e.g. wheeled in at a later time to the same patient bed, or could be at a different physical location, e.g. the patient has been moved to a different ward or care unit and imaging is recommenced at the new location.

The method further comprises acquiring live ultrasound image data of the patient anatomy at the second ultrasound imaging device 122.

The method further comprises registering a current image of the live ultrasound image data with the reference image. The method further comprises, based on the registration, providing acquisition guidance on the user interface 124 to guide a user in adjusting the acquisition of the live ultrasound imaging data to improve a match between a view represented in the current image and a view represented by the reference image.

For example, the method may further comprise recurrently generating a live similarity measure indicative of a similarity between the view represented in the reference image data and a view represented in the live ultrasound image data. In other words, as the operator of the second ultrasound imaging device is acquiring imagery of the patient, a similarity measure is concurrently calculated in the background, indicative of the degree to which the view that is being captured matches that of the reference ultrasound data acquired by the operator of the first ultrasound imaging device 114. This has the aim of maximizing consistency in the views of the ultrasound image data acquired at the two different time points.

The method may further comprise generating a user output alert 126 when the similarity measure matches at least one pre-defined criterion, e.g. the similarity meets or exceeds a minimum similarity threshold. This indicates to a user that they have found the correct view and can stop adjusting the probe position. In other examples, the similarity measure might simply be communicated to the user through a user interface device, e.g. displayed on a display device of the ultrasound imaging device 122.

The method further comprises capturing and storing one or more frames of the live ultrasound image data. Optionally, this may be tagged with the patient identifier information. This may be stored in the image datastore 118, optionally tagged with the patient identifier information.

A further example system and computer-implemented method in accordance with at least one set of embodiments will now be outlined in detail, with reference to Fig. 8.

In this example, the system includes a patient monitor 132, and wherein the datastore 118 previously mentioned is comprised by the patient monitor 132. The patient monitor also includes a user interface 134. The datastore corresponds to the image datastore 56 previously discussed with reference to Fig. 2.

According to this example, a longitudinal patient imaging and monitoring workflow is implemented in which the concepts of the invention are applied to improve consistency of imaging over extended monitoring periods.

According to this particular example, the system permits bi-directional communication and information flow between each of one or more ultrasound imaging apparatuses 114, 122 comprised by the system, and the patient monitor 132. This facilitates imaging, analysis, and patient monitoring workflows involving multiple users (for example including clinical experts and non-experts) and potentially multiple ultrasound platforms (e.g. cart-based ultrasound imaging console, mobile ultrasound imaging probe, or smartphone or tablet computer based devices). In other words, a plurality of different types and classes of node device can connect to the distributed computing system and contribute to a common patient workflow. The system of this set of embodiments enables a multi-stage workflow described below. In general, the principles of the computer-implemented method which is performed are most advantageously applicable for cases where longitudinal monitoring is needed (e.g., lung monitoring, or monitoring of any other anatomical structure). Longitudinal means over an extended period which may be a continuous extended period, e.g. hours of monitoring, or an extended period with one or more interruptions, e.g. where the patient is moved between different care units etc. It in general means monitoring over a period longer than a single ultrasound examination event.

The data flow of the method according to the embodiment of Fig. 8 may, in more detail, be as follows. The data flow can be understood as comprising two phases. A first phase comprises initial patient evaluation by a first (e.g. expert) user of a first ultrasound imaging apparatus. The second phase comprises a subsequent patient evaluation by a second user, who may be a non-expert user of the ultrasound imaging apparatus and/or not as clinically skilled as the first user.

With reference to Fig. 8, in the first phase, the workflow and data flow may be as follows.

When the patient 112 gets an ultrasound evaluation for the first time (at time t₁), it is assumed to be by an expert user skilled in the use of ultrasound. The expert user evaluates the patient using the first ultrasound imaging device 114 (e.g. TTE or TEE imaging). The user acquires all of the views known to be clinically relevant and required. The user may select views that yield the most clinically useful images for that patient, and either mark or tag these images, or delete the other images which are not part of the subset selected as most clinically useful. The image data may in one or more other ways be annotated, edited or pre-processed by the first expert user. The user may label one or more images or subsets of images according to a view which they represent. In other words, the user may curate the images. The user may generate one or more quantitative measurements from the images, manually or using automated algorithms as discussed previously in this disclosure.

The curated images and/or measurements acquired by the first user at the first ultrasound imaging device 114 are transferred to the patient monitor subsystem 132, where they are stored in a datastore 118 comprised by the patient monitor subsystem. The patient monitor subsystem may include one or more client patient monitor devices, which may include one or more bedside patient monitor devices, and may include a central patient monitor console to which each client device is connected. The central monitoring console may house the datastore 118. The first image data 116 may be displayed on a display of a user interface 134 of the patient monitor subsystem. The patient monitoring system optionally may at the same time be arranged to receive biological sensor data relating to the patient 112, and adapted to display this on a display of a user interface.

The display of the user interface 124 may also be controlled to display the acquired first ultrasound imaging data.

In a variation, instead of the image data 116 being transferred to the patient monitor subsystem for storage, a bridge processing module may be provided (not shown), which is connected to a patient monitor subsystem 132, and wherein the bridge processing module comprises the datastore 118. For example, the bridge processing module may be communicatively accessible to both the patient monitor subsystem and each of the one or more ultrasound imaging devices 114, 122 which are comprised by the system.

In addition to acquiring the image data, the first user may optionally also input at the first imaging device 114 one or more imaging recommendations, each indicative of one or more recommended views or images to be acquired at a specified subsequent time point. The recommendation may be generic in terms of its timing, i.e. a recommendation simply for a next imaging acquisition event. Alternatively, the timing may be specific, e.g. recommended view(s) for each of one or more specific future time points, tₙ. This recommendation might for instance be based on the expert user's experience during this first acquisition with respect to the best available views, acoustic windows, specifics of the patient anatomy and so on.

The generated recommendations may be transferred to the datastore 118 along with the acquired first image data, for storage in a data record that is associated with the patient. For example, the image data and recommendation(s) may each be tagged with patient identifier information so that it can later be accessed by querying the database with the patient identifier information.

The second phase is a subsequent ultrasound examination by a further user, who may be a non-expert user, not very familiar with ultrasound.

At a subsequent time point, when ultrasound imaging is deemed necessary for the patient, a second user of the system can use any second ultrasound device 122 connected to the system (of which there may be multiple, in different physical locations) to image the patient. The ultrasound imaging device 122 used to image the patient on the second occasion may be the same or different to the ultrasound imaging device 114 used to acquire the first image data.

By way of example, the user at the second imaging device 122 could input patient identifier information to initiate the device for use with the patient. The ultrasound device then, either automatically, or upon prompt by a user through input at a user interface, communicates with the datastore 118 to retrieve stored image reference image data for the patient. The datastore is for example queried using the patient identifier information. In the present case, the first image data 116 that was stored in the datastore 118 by the expert user is found and is retrieved. All of the image data in the datastore 118 may be retrieved or just a subset of it. For example, the user may indicate the target image-based operation which is to be performed using the acquired images, and a subset of the first image data 116 is retrieved comprising one or more images most suitable for performing the relevant image-based operation. For example, each image of the first image data may be labelled in advance, at a time point between acquiring the first image data and acquiring the second image data, with one or more suitability scores, each indicative of suitability of the relevant image for performing the image-based operation.

For example, the user may indicate the particular examination or monitoring protocol that they plan to implement, and the most appropriate images therefor may be retrieved. The retrieved images are transferred from the datastore 118 to the second ultrasound imaging device 122.

Where the first user also provided view recommendations, these may also be transferred to the second ultrasound imaging device 122. In some examples, these may be communicated to the second user by means of a user interface 124 of the ultrasound imaging device. In some examples, the view recommendations may be utilized to determine which of potentially a plurality of different image views stored in the datastore 118 associated with the patient are transferred to the second ultrasound imaging device.

For example, the datastore 118 may store a set of reference images depicting different respective views of the anatomy, each tagged in accordance with the view depicted, and wherein the datastore further stores the aforementioned view recommendation indicative of a recommended next view of the anatomy to capture, and wherein the reference image retrieved and sent to the second ultrasound imaging device 122 is one of the set of images depicting the recommended next view.

The second ultrasound imaging device 122 then uses the ultrasound image data retrieved from the datastore 118 as reference ultrasound image data for use in guiding acquisition of a second set of ultrasound data, in the manner previously discussed. For example, the ultrasound imaging device 122 may provide, via the user interface, acquisition guidance to the second user to match the current live image to the reference image(s).

In some examples, the acquisition assistance can be in the form of view recognition, wherein live ultrasound image data being acquired by the second user is analyzed in real time, based on registration of the live image to the reference image data, to determine a live similarity measure between the view represented in the reference image data and a view represented in the live ultrasound image data. The user interface 124 of the second ultrasound imaging device 122 may optionally be controlled to generate a user output alert 126 when the similarity measure matches at least one pre-defined criterion, for example, when a certain threshold has been met for the similarity measure.

Additionally or alternatively the acquisition assistance can be in the form of active probe guidance, wherein a processor of the ultrasound imaging device generates user guidance instructions for guiding movement of the probe so as to improve a similarity measure between the view in the live image data and a view represented in the reference image.

The method further comprises capturing one or more frames of the live ultrasound image data and storing the imaging data in the datastore, optionally tagged with the patient identifier information, and optionally tagged with the acquisition time of the data. In some cases, the ultrasound image data may be captured from the second imaging device 122 only when the similarity measure meets said pre-defined criterion. In some cases, the method comprises capturing and storing said at least one frame of the live image data only when the similarity measure meets said pre-defined criterion, and only responsive to a capture command input by a user from a user input device 124. In other words, the user controls the exact timing of image data capture, but wherein the possible timings are constrained according to the similarity measure between a live view of the imaging device and a view of the reference imaging data meeting the pre-defined criterion.

Each of the first 114 and second 122 ultrasound imaging devices, and the patient monitor subsystem, mentioned above comprise a processing arrangement comprising one or more processors which may implement one or more of the steps of the method outlined above.

In some cases, the method may further comprise processing the captured at least one frame of the live image data with an anatomical measurement algorithm, to obtain one or more anatomical or physiological measurements. This processing may be done at the ultrasound imaging device 122 itself, or may be done elsewhere, for example at the patient monitor subsystem 132.

To summarize, the workflow of the particular embodiment of Fig. 8 may be outlined as follows. First ultrasound image data 116 for a given patient, n, is acquired at one ultrasound imaging device of the system, e.g. by an expert user, at a first time *t₁*. The user may annotate and curate the images. Measurements may be generated from the images. The curated images and/or measurements from the initial expert user acquisition are transferred to the patient monitor 132, preferably along with a recommendation for the desired views and/or images to be acquired for the same patient, n, at a next time point, or at a specified future time point, *tₙ*. At a subsequent time point, for instance when ultrasound imaging is deemed necessary for the patient, or in accordance with a timing specified in the recommendation, the system retrieves the first images 116 for the patient from a database in the patient monitor datastore 118 and transfers them to the current ultrasound system being used as reference images. The system identifies an at least subset of the first images 116 which meet a suitability criterion for performing a defined image-based operation, as discussed above. The system then uses the transferred image(s) as the reference image(s) and provides acquisition assistance to the user with respect to the reference image(s).

In accordance with any of the examples or embodiments of the system and computer-implemented method outlined above, there may be further components or devices comprised by the system. For example, the system may further comprise one or more mobile computing devices (e.g. smartphones or tablet computers) which are communicatively connected, linked or networked with the other components of the system.

In accordance with any of the examples or embodiments of the system and computer-implemented method outlined above, there are various options with regards to the data flow between components of the system. In general, it is to be understood that the method is for implementation by a distributed computing system, and that therefore functions or method steps recited as being implemented by one particular component may in fact, in other embodiments, be implemented by a different component.

For example, in the above-described embodiment, it was noted that annotation data (e.g. notes, labels, tags) may be input by the first user at the first ultrasound imaging device 114, and be sent for storage at the datastore 118 along with the first ultrasound imaging data 116. However, additionally or alternatively, in further embodiments, annotation data associated with the first image data 116 may instead be input by a first user, and/or by a further one or more users, at a different one or more devices or nodes of the system and sent to the datastore 118 for storage. For example, this information could be input at a user interface 134 of the patient monitor subsystem 132 instead of at the first ultrasound imaging device 114. For instance, the patient monitor subsystem may include a central console with a user interface, and wherein the annotation or measurement information may be input at the central patient monitor console device. Additionally or alternatively, the patient monitor may include one or more peripheral (bed-side) monitoring devices and the annotation and/or measurement information may input at a user interface of one or more of these peripheral devices. In some embodiments, the system may further comprise one or more mobile computing devices (e.g. smartphone or tablet computer) communicatively coupled or linked with the datastore 118, and wherein annotation and/or measurement data is input by one or more users at one or more mobile computing devices for transfer to the datastore 118, wherein these one or more users may or may not include the first user (e.g. a different expert user provides the annotations).

Furthermore, the annotation or measurement information may be input at the same time or a different time to the image data acquisition, e.g. later on a mobile device of the first user or different user, and transferred to the datastore for storage.

Furthermore, according to one or more embodiments, the first user acquiring the first ultrasound image data may input at the first ultrasound imaging device an indicated selection of the acquired first ultrasound images which should subsequently be used as a reference image for a subsequent image data acquisition, and this information transferred to the datastore 118 for storage. This information may be input by the first user at the first ultrasound imaging device 114, or may be input (by the same first user or a different one or more users) at a further one or more devices or nodes of the system.

Furthermore, the system may include functionality to permit the first user who acquires the first ultrasound image data 116, and/or another user of the system, to input a recommendation for a time at which subsequent ultrasound image data should be acquired.. For instance, and by way of illustration, an expert clinician reviewing a patient in person may review existing ultrasound images for the patient for instance using a user interface 134 of a patient monitor subsystem 132, and these being retrieved from the datastore 118, and may decide that another ultrasound acquisition would be clinically valuable, acquired in two hours' time. The patient monitor device (or another device of the system, such as a mobile computing device) may permit input by the user at a user interface of an instruction signal by which a future ultrasound imaging event is scheduled, ordered, or recommended, including a specific time for ultrasound imaging acquisition. Optionally, a message might be generated and pushed to one or more further devices of the system, to alter the user(s) of the one or more further devices that the new ultrasound image acquisition has been ordered. Any user (expert or non-expert) can then acquire the recommended subsequent ultrasound imaging data at the specified time, using the guidance functionality which has been described above.

The above description represents just one example set of embodiments, not all features of which are essential to the inventive concept.

For example, instead of using the patient monitor to store the database of patient image data, annotation data, measurement data and/or further data, this data might be stored on a different module such as an interface module (having a datastore comprised thereby) connected between one or more of the ultrasound imaging devices and the patient monitor device.

In general, the various devices and components comprised by the system may be spatially separated from one another, for example distributed at different locations across a medical institution. They may be communicatively connected, linked or networked with one another via one or more network connections. Their communication with one another may be facilitated by one or more components acting as network hubs or servers. Communication between them may be facilitated though a local or wide area network for example, and/or by use of an internet connection. For example, each device might be operable to connect, through a login facility, to a common internet web portal, via which interconnection between the device is facilitated.

Furthermore, it is noted that although 'first' image data and a 'first' user are referred to above, this for convenience of reference and concision only. It is not essential that the ultrasound data referred to as 'first' ultrasound data is in fact the very first ultrasound data acquired for the given patient *n.* The descriptions above intend to merely reflect the concept that one set of image data can be acquired at any of a potential plurality of ultrasound imaging devices of the system, and that one or more images from this data can then be used as a reference for use in acquiring subsequent image data for the same patient at another or the same of the potential plurality of ultrasound imaging devices.

Various embodiments outlined above refer to generating processing ultrasound image data with an anatomical measurement algorithm to derive one or more anatomical or physiological measurements, e.g. hemodynamic measurements. For example, one or more embodiments may comprise processing the captured at least one frame of the live image data with an anatomical measurement algorithm. In one or more embodiments, the first images captured at the first ultrasound imaging device 114 may be processed with an anatomical measurement algorithm to derive one or more anatomical or physiological measurements.

By way of example, the measurement algorithm may employ use of model based segmentation (MBS) to segment boundaries of one or more anatomical structures and to thereby derive dimensional measurements and/or functional measurements such as movement patterns or fluid flow measurements.

For example, for cardiac imaging ultrasound imaging can be used for obtaining quantitative measurements of hemodynamic parameters such as left ventricular (LV) volume, stroke volume (SV), cardiac output (CO), LV Ejection Fraction (LVEF). A trend pattern of these parameters can be tracked over time and used to inform patient treatment.

It is known for example to apply model-based segmentation to acquired ultrasound imagery in order to obtain quantitative measurements For example, automatic segmentation of one or more chambers of the heart can be performed. Measurements of hemodynamic parameters can be obtained through applying model based segmentation on an end-diastolic (ED) ultrasound image frame to detect heart chamber boundaries, followed by 2D or 3D tracking of the segmentation boundaries over the other frames of the cardiac cycle.

For 2D imaging, geometric assumptions can be made to extrapolate from acquired single-plane or bi-plane information, to generate measurement information for 3D objects or features, e.g. heart chamber volume. Example algorithms include modified Simpson's rule, ellipsoidal model using single-plane, bi-plane or unidimensional data, or the hemisphere-cylinder model based on bi-plane data.

For further details on these segmentation methods, reference is made to: Kosaraju A, Makaryus AN. Left Ventricular Ejection Fraction. In: StatPearls [Internet]. Treasure Island (FL): StatPearls Publishing; 2019 Jan-. Available from: https://www.ncbi.nlm.nih.gov/books/NBK459131/.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A method for ultrasound image acquisition guidance, comprising:
obtaining an indication of a target image-based operation to be performed during an ultrasound scan with an ultrasound imaging apparatus;
identifying, in an image datastore, a reference image associated with the target image-based operation to be performed, the reference image having been acquired in a previous ultrasound scan with the same or a different imaging apparatus, and wherein the identifying comprises identifying the reference image as meeting a pre-defined suitability criterion indicative of suitability for performing the target image-based operation;
acquiring live ultrasound image data at the ultrasound imaging apparatus;
registering a current image of the live ultrasound image data with the reference image; and
based on the registration, providing acquisition guidance on a user interface to guide a user in adjusting the acquisition of the live ultrasound imaging data to improve a view represented in the current image.

2. The method of claim 1, wherein improving a view represented in the current image comprises improving a match a view represented in the current image and a view represented in the reference image.

3. The method of claim 1 or 2,
wherein the reference image is labelled in the datastore with a suitability score for performing the target image-based operation, and
wherein the suitability score associated with the image is determined by:
(i) reading the suitability score label from the database; or
(ii) applying a suitability analysis module to the reference image configured to generate a suitability score.

4. The method of any preceding claim,
wherein the image datastore stores a plurality of previously acquired images, acquired in one or more previous ultrasound scans, and each previously acquired image labelled with a view represented by the image;
the method comprising:
detecting a view represented by the current image of the live ultrasound image data using a view detection module; and
identifying an image from the image datastore which meets the suitability criterion for the target image-based operation and which represents a matching view to the currently acquired image.

5. The method of claim 4,
wherein each image in the datastore is associated with a suitability score for performing the target image-based operation and wherein the identifying the image from the image datastore comprises identifying an image from the image datastore having the highest suitability score for the target image-based operation and which represents a matching view to the currently acquired image; and
preferably wherein at least a subset of the images stored in the image datastore are each labelled with suitability scores for more than one different possible image-based operation.

6. The method of any of claims 3-5, wherein the method comprises computing the suitability scores for the images in the image datastore by application of a suitability analysis module which utilizes a trained convolutional neural network trained using prior images of an anatomy which have been labelled with suitability scores for at least one image-based operation.

7. The method of any preceding claim,
wherein the target image-based operation comprises acquiring an image representing a pre-defined target view of an anatomical structure;
wherein the suitability criterion/score comprises an image quality criterion/score representative of an image quality of the reference image; and
wherein preferably the image quality criterion/score is based on a level of image noise, a level of image blur, and/or a visibility of one or more pre-defined anatomical features.

8. The method of any preceding claim,
wherein the image-based operation comprises acquiring a target measurement of an anatomical structure depicted in the current image; and
wherein the suitability criterion represents a suitability of the reference image for use in acquiring the target measurement.

9. The method of claim 8, wherein performing the image-based operation comprises applying a measurement quantification module to the current image of the live ultrasound image data to acquire the target measurement from the current image.

10. The method of any preceding claim, wherein the providing the acquisition guidance comprises:
displaying an overlay of one or more reference points of the reference image atop the current image, for example wherein the reference points represent anatomical landmarks; and/or
displaying an overlay of a field-of-view boundary of the reference image atop the current image, based on the registration.

11. The method of claim 10,
wherein the providing the acquisition guidance comprises displaying an overlay of a field-of-view boundary of the reference image atop the current image, based on the registration;
wherein the registration comprises determining a rotation offset and a translation offset between an anatomy depicted in the current image of the live ultrasound image data and the same anatomy depicted in the reference image;
wherein the displaying an overlay of a field-of-view boundary of the reference image comprises estimating a location of a field-of-view boundary of the reference image relative to the current image based on the rotation offset and translation offset, and rendering the overlay of the field of view boundary based on the estimate.

12. The method of any preceding claim, wherein the reference image is labelled in the database with a set of acquisition parameters of an ultrasound imaging apparatus used to acquire the reference image, and wherein
the method comprises configuring the ultrasound imaging system based on the set of acquisition parameters used to acquire the reference image; and/or
the method comprises generating a guidance prompt on a user interface indicative of the set of acquisition parameters used to acquire the reference image.

13. A computer program product comprising machine-executable code configured, when run on a processor, to cause the processor to perform a method in accordance with any of claims 1-12.

14. A processing device comprising one or more processors configured to perform a method in accordance with any of claims 1-12.

15. A system comprising:
the processing device of claim 14; and
an ultrasound acquisition apparatus operatively coupled with the processing device.
